# EUROPEAN PATENT APPLICATION

(11) **EP 1 018 323 A1**
(43) Date of publication of application: **12.07.2000**
(21) Application number: 99100115.7
(22) Date of filing: 04.01.1999
(51) Int. Cl.: A61F 5/451

(54) **Container for the collection of bodily waste with an unfolding means**

(71) Applicant: THE PROCTER & GAMBLE COMPANY, Cincinnati, Ohio 45202 (US)
(72) Inventor: Cinelli, Fabio, 40133 Bologna (IT); Colaianni, Antonello, 65126 Pescara (IT); Evangelista, Olindo, 66023 Francavilla al Mare, Chiete (IT)
(74) Representative: Kohol, Sonia

(57) **Abstract**

The present invention relates human waste management device, such as urine management devices and faecal management devices, these devices comprising a bag for storage of human waste and being suitable for babies, children or adults. The invention resides principally in providing such devices with a means to unfold the bag (11). One preferred means is a string attached to the bag (11).

## Description

### Field of the invention

The present invention relates to urine management devices and to faecal management devices, all comprising storage means, such as a bag, and all being suitable for babies, children or adults. Said devices are provided with a means to unfold the bag, which typically is supplied in a folded configuration prior to use.

### Background of the invention

Human waste management devices are known articles of manufacture that are designed to be worn principally by incontinence sufferers and in particular by bedridden patients. Such human waste management devices are attached to the perianal or uro-genital region of the wearer and are intended to entrap and immediately contain faecal material, urine and other bodily discharges. Such devices, as they are mostly known today are constituted of a bag at one extremity of which is positioned the aperture and the attachment device, which typically is adhesive.

For example faecal management devices are disclosed in the following documents: US 3,577,989, which details a disposable plastic bag for incontinence sufferers. US 4,784,656 describes a receptacle for collecting faecal matter. The receptacle is formed from two sheets of thermoplastic film that are heat sealed along their side edges. GB 2 152 387, which teaches a faecal collector for incontinence sufferers comprising a collection bag consisting of a pair of panels of thermoplastic sheet material joined at their margins.

EP 245 064 discloses bags substantially consisting of a front and a rear wall, which are made of a synthetic plastic material, such as PVC. GB 2 215 605 discloses an ostonomy bag comprising panels of synthetic plastic material, the rear bag wall further comprising a needled film.

Urine management devices are, for instance, disclosed in the following documents: GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. GB 2,268,882 discloses a urostomy pouch/bag of plastic material provided with a circular stomal orifice which is surrounded by a first coupling member, by which the pouch can be affixed to a counterpart coupling member, which can be attached to a wearer. US 4,804,377 discloses a collector for urine specimens from children. The collector comprises a rectangular flange for adhesive attachment, which comprises a round, slightly oval aperture. EP 140 478 discloses a disposable diaper having a water proof barrier preferably polypropylene or polyethylene formed as a flattened bag having a single opening. US 1,092,274 and US 3,292,626 disclose a urine collector for female infants. Chinese patent application CN 1079381 discloses urine bags for infants with circular and elliptical apertures.

All human waste management devices disclosed in the documents, which are referred to above, are typically used for bedridden patients, independent of whether they are attached to the uro-genital area or the natural or an artificial anus. Thus they are typically stored in a hospital and applied by a trained caretaker, for example a nurse. However, in a recent patent application (EPO application 97110603.4; "Shaped faecal management device") and co-pending applications, faecal management devices are disclosed which are optimised also in respect to the use for babies. Such use encompasses that the human waste management device is stored, transported and carried in many ways, for example a mother may want to carry one or more devices in her handbag. Therefore it is desirable to provide the human waste management device in a small and handy format. Typically this is achieved by folding the bag (11). For application to a baby ideally the mother has one hand available for the baby, e.g. to hold up the legs and the other to apply the device. Moreover, the application should be quick and convenient, since it is carried out in a large variety of circumstances. It is also desirable that the application of the device is self-explanatory and can be carried out intuitively by an untrained person.

Hence, there still exists a need for human waste management devices which can be quickly and easily placed and handled, preferably using only one hand.

It has now been found, that all of the above outlined problems can be addressed by providing a human waste management device with an unfolding means, such as a string.

### Summary of the invention

The present invention relates to human waste management device suitable for use for babies, children or adults. The invention resides principally in providing such devices with an unfolding means, such as a string.

### Brief description of the drawings

It is believed that the invention will be better understood from the foregoing description in conjunction with the accompanying drawings in which:
Figure 1 is a perspective view of a preferred faecal management device (10) according to the present invention.
Figure 2 is a cross sectional view of the preferred faecal management device (10) shown in Figure 1.
Figure 3 is a cross sectional view of another preferred faecal management device (10) according to the present invention which comprises an applicator (40).

### Detailed description of the invention

The invention relates to human waste management devices. Such a human waste management device may be a faecal management device (10), which is designed for attachment to the anal area and mainly used for collecting faeces, or it may be a urine management device, which is attached to the urinary duct and mainly used for collecting urine. A human waste management device may also be a device to collect both urine and faeces and thus be attached to both of the above areas. All of the above human waste management devices are preferably designed for single use and disposal thereafter.

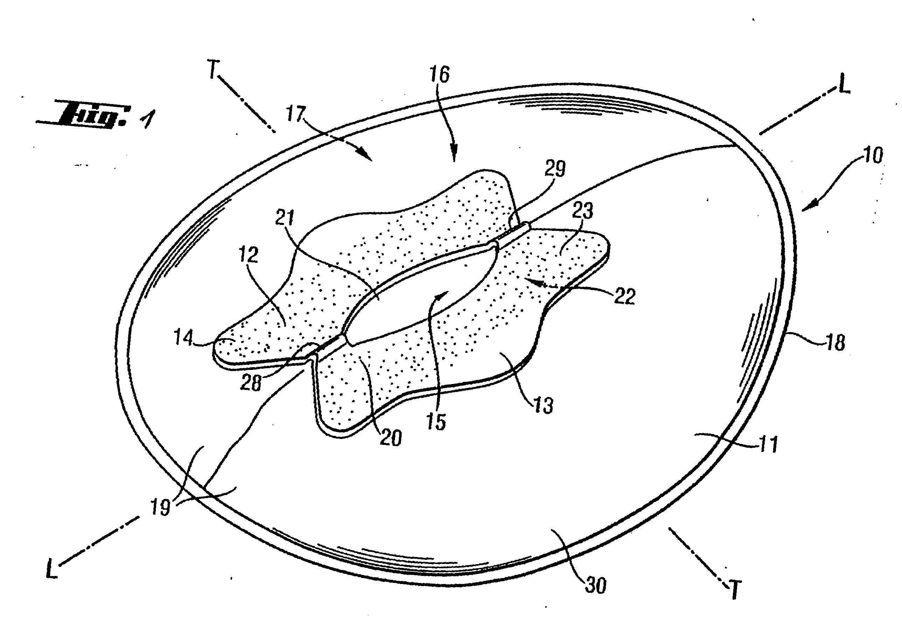

A faecal management device (10) is shown in Figure 1.

### Description of the human waste management device as a whole

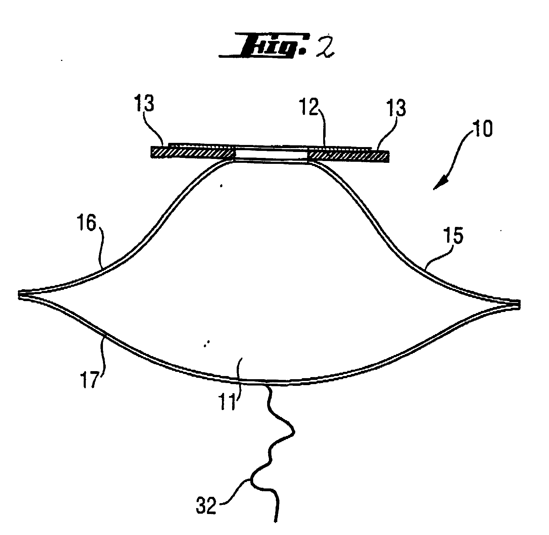

Typically human waste management devices comprise a bag (11) having an aperture (21) and a flange (12) surrounding the aperture for preferably adhesive attachment to the perianal area of a wearer as visible from Figure 2. Any human waste management device known in the art can be provided according to the present invention.

The bag (11) as used herein is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

A variety of shapes of the bag is within the scope of the present invention. Particularly, preferred shapes are flat circular type bags, cone shaped bags, truncated cone shaped bags and pyramidal or truncated pyramidal shaped bags and flat T shaped bags.

The *garment facing portion (17)* is the portion of the bag (11), which is generally oriented away from the wearer, when the human waste management device (10) is worn, and towards a garment, if a garment is worn. The garment facing portion (17) does not comprise the aperture (21). The size and the shape of the garment facing portion (17), in particular its length and width, are defined by the bag peripheral rim (18) if present. Sections of material comprised by the rim (18) or seal do not form part of the garment facing portion (17).

According to the present invention the bag material can comprise one or multiple layers, preferably two or three layers. The layer on the inside of the bag (11), which will typically at least partially come in contact with faecal material or urine is called the inner layer. The outermost layer of the bag, which will typically at least partially come in contact with the skin to the wearer and the garments of the wearer, is called the outer layer.

The layers of the bag material may comprise any material, preferably so that the bag is liquid impervious. The preferred optical properties of any such material will be described below in more detail. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag material may comprise a non-woven layer. Such material layers present an uneven surface to the skin of the wearer and thus reduce significantly the problem of occlusion and greatly improve skin healthiness.

In one preferred embodiment of the present invention the bag material comprises two layers. Preferably the outer layer comprises a non-woven layer and the inner layer comprises a film.

In yet another preferred embodiment of the present invention, the bag material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag material may be hydrophobic or hydrophilic. If the bag material does not comprise a film layer, preferably at least one non-woven layer is hydrophobic. As a consequence, fluid penetration is resisted through the wearer facing portion (16) and the garment facing portion (17) of the human waste management device (10). If the bag material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

The bag (11) is provided with an aperture (21) whereby faecal matter or urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (13).

The flange comprises a garment facing portion (22) and a wearer facing portion (23). In an preferred embodiment these are two large, substantially flat surfaces, however, the flange (12) may also comprise projections, a front projection (28) and/or a rear projection (29), in case of a faecal management device (10) designed to fit the perineal and/or coccygeal area of the wearer and in case of a urine management device (10) designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

According to the present invention the human waste management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 2, the adhesive is in one preferred embodiment not applied to the entire wearer facing portion (23) of the flange (12), so as to provide lobes (13) on either side of the flange (12) which are non-adhesive and can thereby serve to facilitate placement and removal of the device whilst avoiding contact with the adhesive. These lobes (13) are however preferably also covered by the release means. Before application of the human waste management device (10) to the skin of the wearer, the release means if present is removed.

According to the present invention any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste management devices (10) to be used for babies the amount of adhesive (20) may be less than for human waste management devices (10) designed for active adult incontinence sufferers.

The human waste management device (10) of the present invention has been found to be particularly useful and beneficial when used in conjunction with a garment, or diaper (50), preferably a disposable diaper.

### Detailed description of the unfolding means

According to the present invention a human waste management device, such as a faecal management device (10) or a urine management device, which comprises a bag (11) as a storage means for human waste, is provided in a prior to use configuration in which the bag (11) is typically folded and with a means to unfold the bag (11).

Within the scope of the present invention the unfolding means may be any device which can be attached to the bag (11) and allows easy gripping by human fingers or attachment to an applicator (40).

While the present invention comprises urine management devices as well as faecal management devices the following description focuses on faecal management devices. However, the person skilled in the art has no difficulties to apply the present invention to a urine management device.

One preferred embodiment of the present invention is shown in Figure 2. There a faecal management device (10) is provided with a string (32) as a means to unfold the bag (11). The string (32) is attached to the garment facing portion (17) of the bag (11) in a position close to the centre of the garment facing portion (17). When the bag (11) is provided in a folded configuration prior to use, the bag (11) may be unfolded by simply pulling the string (32). This unfolding results in a configuration of the bag (11) which provides more wearing comfort and which also ensures that the bag (11) provides sufficient volume to contain human waste. To the same benefit the string (32) may be used if the bag (11) is provided in any other prior to use configuration which is different from the intended wearing configuration.

The string (32) may be provided from any material such as natural fibres, e.g. wool, or artificial fibres or a plastic material. The string (32) may be attached to the bag (11) by any means known to man skilled in the art such as stitching, thermobonding, ultrasonic bonding, adhesive and a hook/loop ensemble such as Velcro®.

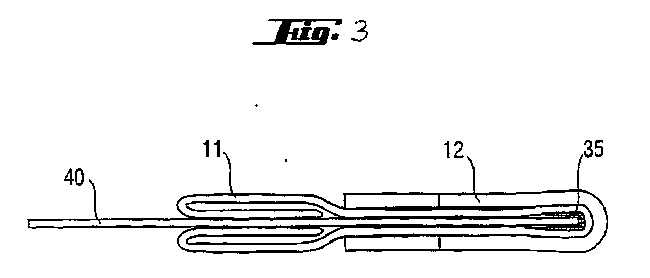

To ensure convenient packaging and transport the bag (11) is typically provided in a folded configuration prior to use. While any folding pattern is within the scope of the present invention, one configuration is shown in Figure 3. It is however to be understood that the bag (11) may also be provided with such a folding pattern, when the human waste management device is not provided with an applicator (40). When the bag (11) is provided in a folded configuration the unfolding means should preferably be accessible without partly or even fully unfolding the bag (11). To that benefit at least a portion of the unfolding means preferably extends beyond the prior to use contour of the bag (11). If, for example, a string (32) is used as a unfolding means the length of the string (32) should be chosen so, that portions of the string (32) extend beyond the contour of the folded bag (11).

The faecal management device (10) or urine management device may be provided in combination with an applicator (40), e.g. as described in Figure 3 and in patent application No. PCT/US98/13298, "Applicator for a faecal management device". Preferred unfolding means within the scope of the present invention are also those means which establish a releasable link between the applicator (40) and the bag (11). For example such a link may be provided by releasable adhesive attachment or by a hook/loop ensemble, such a Velcro®.

One preferred embodiment comprising such a link is shown in Figure 3. In this embodiment one portion of the Velcro® ensemble (35) (e.g. the one comprising loops) is attached to the garment facing portion (17) of the bag (11) while a corresponding portion of Velcro® (35) (e.g. the one comprising hooks) is attached to the applicator (40). After attachment of the human waste management device to the wearer the applicator (40) is usually detached from the device (10), typically by pulling it away from the wearer. The action of pulling it away from the wearer will in this embodiment automatically lead to the unfolding of the bag (11) due to the link provided by the Velcro® ensemble (35): When the bag (11) is fully unfolded further pulling of the applicator (40) in the direction away from the bag (11) will be hindered by increased forces transmitted by the now unfolded bag (11). These increased forces will lead to the separation of the Velcro® ensemble (35), whose separation forces are to be appropriately chosen. Thus, the unfolding of the bag (11) does not require any handling step further to the application of the device by an applicator (40) and the removal of the applicator (40).

## Claims

1. Human waste management device comprising a bag (11) characterised in that said human waste management device comprises an unfolding means.

2. Human waste management device according to claim 1 wherein said bag (11) comprises said unfolding means.

3. Human waste management device according to Claim 2 wherein said bag (11) comprises a garment facing portion (17) and a wearer facing portion (16) and said garment facing portion (17) comprises said unfolding means.

4. Human waste management device according to any one of the preceding claims wherein said unfolding means comprises a string (32).

5. Human waste management device according to any one of the preceding claims wherein said unfolding means comprises a hook/loop ensemble (35).

6. Human waste management device according to any one of the preceding claims wherein said bag (11) is folded prior to use.

7. Human waste management device according to Claim 6 wherein said folded bag (11) defines an outer contour and at least a portion of said unfolding means extends beyond said outer contour of said folded bag (11).

8. Human waste management device according to any one of the preceding claims wherein said device further comprises an applicator (40).

9. Human waste management device according to Claims 5 and 8 wherein said applicator (40) comprises a portion of said means to unfold the bag (11).
